# EUROPÄISCHE PATENTSCHRIFT

(11) **EP 0 894 870 B1**
(45) Veröffentlichungstag und Bekanntmachung des Hinweises auf die Patenterteilung: **10.10.2012**
(21) Anmeldenummer: 98103296.4
(22) Anmeldetag: 13.08.1996
(51) Int. Cl.: C12Q 1/68

(54) **Extraktion von Pilzzellen-DNA aus klinischem Material**
Extraction of fungal cells from clinical material
Extraction d'ADN de cellules de champignons d'un materiel clinique

(30) Priorität: 17.08.1995 DE 19530332; 17.08.1995 DE 19530333; 17.08.1995 DE 19530336
(43) Veröffentlichungstag der Anmeldung: 03.02.1999
(62) Teilanmeldung aus: 96929253.1
(73) Patentinhaber: Myconostica Limited, Sharston Manchester M22 4SN (GB)
(72) Erfinder: Einsele, Hermann, Dr., 72076 Tübingen (DE)
(74) Vertreter: Trinks, Ole

(56) Entgegenhaltungen:
- EP-A- 0 285 439
- EP-A- 0 574 267
- WO-A-92/08807
- WO-A-93/23568
- US-A- 4 130 395
- US-A- 5 426 026
- US-A- 5 426 027
- HOLMES ET AL.: "Detection of C. albicans and other yeasts in blood by PCR" JOURNAL OF CLINICAL MICROBIOLOGY, Bd. 32, Nr. 1, Januar 1994, Seiten 228-231, XP000645486 WASHINGTON, US

## Beschreibung

Die vorliegende Erfindung betrifft allgemein ein Verfahren zum Nachweisen von Pilzzellen in klinischem Material unter Extrahieren von Pilz-DNA aus Vollblut mit den Schritten:
a) Isolation überwiegend intakter Pilzzellen aus dem Vollblut, und
b) Extrahieren von DNA aus den isolierten Pilzzellen durch
b1) Aufschließen der isolierten Pilzzellen, und
b2) Isolation der Pilz-DNA,
wobei der Schritt a) die weiteren Schritte umfasst:
a1) Aufschließen der im Vollblut befindlichen Blutzellen; und
a2) Isolation überwiegend intakter Pilzzellen von zellulärer DNA,
wobei der Schritt b1) den weiteren Schritt umfasst:
b1.1) alkalisches Lysieren und enzymatische Behandlung der Pilzzellen, wobei der Schritt a) die weiteren Schritte umfasst:
a1.1) Lysis der roten Blutkörperchen durch osmotische Hämolyse;
a1.2) enzymatisches Aufschließen der weißen Blutkörperchen; und
a2.1) Zentrifugation des so behandelten Vollblutes und Verwendung des Pellet in den nächsten Verfahrensschritten,
und wobei der Schritt b1.1) folgende Schritte umfasst:
- Inkubieren der im Pellet aus Schritt a2.1) befindlichen Pilzzellen bei 90-95°C, vorzugsweise 95°C für 5-15 min, vorzugsweise 10 min, in einer Lösung mit 50 mM NaOH, und
- Neutralisation mit 1 M Tris-HC1 pH 7,0,
- enzymatische Behandlung mit Zymolase für 50-70 min, vorzugsweise 60 min bei 30-40°C, vorzugsweise 37°C,
- Proteindenaturierung durch Inkubation mit Tris/EDTA bei 60-70°C, vorzugsweise 65°C für 10-30 min, vorzugsweise 20 min.

Ein derartiges Verfahren ist aus der WO 93/23568 bekannt.

Verfahren zum Nachweisen von Pilzzellen in klinischem Material sind außerdem aus der Praxis bekannt, sie beruhen standardmäßig auf einer Anzüchtung von Pilzspezies aus klinischem Material auf entsprechenden Nährmedien.

Durch diese Anzüchtung z.B. in Petrischalen und den Nachweis anhand der gewachsenen oder auch nicht gewachsenen Kolonien sind die Nachweisgeschwindigkeit sowie -empfindlichkeit insbesondere wegen des langsamen Wachstumes der Pilzspezies stark beeinträchtigt. Die Diagnose der invasiven Pilzinfektion wird daher häufig erst durch eine äußerst komplikationsträchtige Biopsie eines Organes oder sogar erst nach dem Tod des Patienten gestellt.

Das Interesse an Verfahren zum Nachweisen von Pilzzellen ist vor dem Hintergrund zu sehen, daß insbesondere in den letzten Jahren Pilzspezies als bedeutende nosokomiale Pathogene eine erhebliche Bedeutung für immunsupprimierte Patienten erlangt haben. Vor allem nach Knochenmarkstransplantationen (KMT), aber auch nach Leber-, Nieren-, Pankreas-, Herz- und Herz-Lungen-Transplantationen haben invasive Pilzinfektionen erheblich zugenommen. So ist es z.B. im Jahr 1994 vor allem in französischen KMT-Zentren zu einer solchen Häufung vor allem von Aspergillusinfektionen gekommen, daß diese Zentren mehrere Monate geschlossen werden mußten.

Neben den organtransplantierten Patienten sind aber auch Patienten mit Krebserkrankung, vor allem nach Chemotherapie oder chirurgischen Eingriffen, Verbrennungspatienten sowie Patienten auf chirurgischen und neonatalen Intensivstationen zunehmend von invasiven Pilzinfektionen betroffen. Sobald es bei diesen Patientenkollektiven zu einer Beteiligung eines Organsystemes oder gar mehrerer Organsysteme kommt, beträgt die Sterblichkeit an dieser infektiösen Komplikation zwischen 80 und 100 %.

Nur durch eine frühzeitige Diagnose kann hier der Behandlungserfolg verbessert werden. Wegen der mit den eingangs erwähnten Standardnachweisverfahren verbundenen Nachteile werden intensive Bemühungen unternommen, eine frühzeitige Sicherung der Diagnose einer systemischen Pilzinfektion zu ermöglichen.

Ein auf molekularbiologischen Techniken beruhendes Verfahren zum Nachweis und zur Differenzierung pathogener Pilzspezies in klinischem Material ist in der eingangs genannten WO 93/23568 beschrieben. In diesem Verfahren werden Pilzzellen aus Patientenblut isoliert, wobei die Pilzzellen zuerst von dem übrigen Blutmaterial abgetrennt werden. Danach wird die Pilzzellwand aufgebrochen und die Pilz-DNA isoliert. Daraus wird dann ein Segment aus dem 5S rRNA-Gen in einer Polymerase-Kettenreaktion amplifiziert und mit verschiedenen Methoden nachgewiesen. Anhand der isolierten DNA können außerdem verschiedene Pilzspezies voneinander unterschieden werden.

Die wesentlichen Probleme des Nachweises von Pilzinfektionen mit Hilfe molekularbiologischer Techniken sind dabei auf die sehr komplexe Zusammensetzung der Pilzzellenwand zurückzuführen, die bisher zeitaufwendige aber auch teure Extraktionsverfahren erforderlich machen.

Ein weiteres Problem besteht darin, daß eine zunehmende Zahl von Pilzspezies bei den immunsupprimierten Patienten bedrohliche Infektionen auslösen kann, woraus die Notwendigkeit resultiert, eine Fülle von verschiedenen Pilzgattungen und -stämmen bei diesen Patienten erfassen und bestimmen zu müssen. Da sich die Therapie der Infektionen bei verschiedenen Pilzspezies nämlich unterscheidet, ist es folglich erforderlich, nicht nur alle Pilzspezies erfassen, sondern diese auch differenzieren und identifizieren zu können.

Vor diesem Hintergrund ist es Aufgabe der vorliegenden Erfindung, das eingangs erwähnte Verfahren dahingehend weiterzubilden, daß eine frühzeitige Diagnose einer Pilzinfektion möglich wird.

Das Verfahren soll dabei möglichst schnell und einfach durchzuführen sein, möglichst viele Pilzspezies erfassen und in einer Weiterbildung auch identifizieren können.

Der Schritt a) umfaßt die weiteren Schritte:
a1) Aufschließen der im Vollblut befindlichen Blutzellen; und
a2) Isolation überwiegend intakter Pilzzellen von zellulärer DNA,
wobei Schritt b1) das alkalische Lysieren der Pilzzellen, vorzugsweise mittels NaOH, und eine enzymatische Behandlung der Pilzzellen umfaßt.

Bei den einzelnen Schritten des neuen Verfahrens waren viele Probleme zu überwinden, die sich zum Teil aus der Zusammensetzung der Pilzzellenwand und aus der Tatsache ergaben, daß ein Großteil der Pilzzellen sich nicht im Vollblut frei in Lösung sondern nach Phagozytose in verschiedenen Blutzellpopulationen befindet, vor allem in Granulozyten und Makrophagen.

Das erfindungsgemäße Verfahren wird zwar vorteilhaft in der Diagnostik eingesetzt, um eine Pilzinfektion bei einem Patienten nachweisen zu können, es findet aber auch dort Anwendung, wo Pilz-DNA für anderweitige Weiterverarbeitung benötigt wird.

Mit dem neuen Verfahren kann dafür z. B. Pilz-DNA aus dem Blut von Tieren gewonnen werden, die speziell mit den interessierenden Pilzspezies infiziert wurden. Aus der so gewonnenen Pilz-DNA können z. B. DNA-Sonden herausgeschnitten werden, die für Nachweisreaktionen verwendet oder in Plasmide eingebaut werden können. Es sind dabei Anwendungen im ganzen Bereich der Grundlagenforschung, Diagnostik, Therapeutik, industriellen Gentechnologie etc. denkbar.

Das Verfahren der Extraktion von Pilz-DNA erlaubt es, insbesondere in der Diagnostik auch bei sehr geringen Mengen von Pilzen in dem vorliegenden Vollblut noch zuverlässig Pilz-DNA extrahieren zu können. Ferner kann dieser Verfahrensschnitt schnell und so einfach durchgeführt werden, daß er auch im Klinikalltag und ggf. von angelerntem Personal angewendet werden kann.

Das neue Verfahren besteht sozusagen aus zwei Stufen, wobei in der ersten Stufe Pilzzellen aus dem Vollblut isoliert werden und dann in der zweiten Stufe die DNA aus diesen isolierten Pilzzellen extrahiert wird, um mit Hilfe dieser extrahierten DNA dann Pilzinfektionen nachweisen und ggf. spezifizieren zu können. Dieses zweistufige Verfahren erhöht vor allem die Spezifität, da ggf. störende, anderweitige DNA in dem zweiten Verfahrensschritt nur noch in geringem Maße vorhanden ist.

Außerdem wird eine sehr schnelle und sichere Trennung der Pilz-DNA von zellulärer DNA erreicht. Durch den Aufschluß der im Vollblut befindlichen Blutzellen wird nämlich in dieser Ausgangslösung die zelluläre DNA freigesetzt, woraufhin dann die durch das bisherige Aufschlußverfahren noch nicht oder zumindest noch nicht vollständig aufgeschlossenen Pilzzellen durch schnelle und einfache Verfahren, wie z.B. eine Zentrifugation von der freien zellulären DNA getrennt werden können. Bei dem daraufhin folgenden Aufschließen der so isolierten Pilzzellen kann mit großer Sicherheit davon ausgegangen werden, daß sich keine oder nur noch unerhebliche Mengen an zellulärer DNA in der Lösung befindet. Das im Schritt a1) durchzuführende Aufschließen der Blutzellen muß folglich so erfolgen, daß die Pilzzellen noch nicht lysiert werden. Da die Pilzzellwand deutlich komplexer ist als die Zellwand von Blutzellen, kann dies durch geeignete vorsichtige Aufschlußverfahren sichergestellt werden.

Der Verfahrensschritt a1) hat jedoch noch einen weiteren Vorteil, durch ihn werden nämlich ggf. phagozytierte Pilzzellen wieder freigesetzt, so daß insbesondere für die Diagnostik auch noch sehr geringe Mengen von Pilzen in dem vorliegenden Vollblut extrahiert werden können. Bei dem neuen Verfahren ist es nämlich nicht mehr erforderlich, daß zumindest einige Pilzzellen in dem Vollblut frei in Lösung sind, es reicht völlig aus, wenn einige wenige Pilzzellen nach Phagozytose z.b. in Granulozyten oder Makrophagen vorliegen.

Die der Erfindung zugrundeliegende Aufgabe wird auf diese Weise vollkommen gelöst.

Der Schritt a) umfaßt die folgende schritte :
a1.1) Lysis der roten Blutkörperchen durch osmotische Hämolyse;
a1.2) enzymatisches Aufschließen der weißen Blutkörperchen; und
a2.1) Zentrifugation des so behandelten Vollblutes und Verwendung des Pellet in den nächsten Verfahrensschritten.

Hier ist von Vorteil, daß in den Schritten a1.1) und a1.2) zwar zuverlässig die Blutzellen aufgeschlossen werden, die Pilzzellen selbst jedoch noch nicht beschädigt sind. Durch die folgende Zentrifugation ist dann eine sehr sichere Trennung zwischen der inzwischen freigesetzten zellulären DNA der Blutzellen und Zellbruchstücken der Blutzellen einerseits sowie noch überwiegend intakten Pilzzellen andererseits möglich. Auf diese Weise wird außerdem sichergestellt, daß keine Pilz-DNA verlorengeht, da diese noch in den im Pellet zu findenden Pilzzellen vorhanden ist. Zusammengefaßt liegen die Vorteile der obigen Schritte also darin, daß zum einen auch geringste Konzentrationen von Pilzzellen erfaßt werden und daß zum anderen die isolierte Pilz-DNA allenfalls gering mit zellulärer DNA verunreinigt ist, so daß eine hohe Sensitivität und Spezifität bei den nachfolgenden diagnostischen Schritten möglich ist, da das Verhältnis von Pilz- zu zellulärer DNA zugunsten der Pilz-DNA deutlich verbessert wurde.

Der Schritt b) umfaßt folgende Schritte :

### b1.1) alkalisches Lysieren und enzymatische Behandlung der Pilzzellen.

Es hat sich herausgestellt, daß durch diese einfachen Verfahrensschritte ein sicheres Aufschließen der Pilzzellen möglich ist, die aus dem Pellet des Verfahrensschrittes a2.1) wieder aufgenommen wurden.

Weiterhin ist es bevorzugt, wenn im Schritt a1.1) die Lysis der roten Blutkörperchen mit Hilfe einer hypotonen Lösung erfolgt, vorzugsweise mit einer Endkonzentration von ca. 10 mM Tris pH 7,6, 5 mM MgCl₂, und 10 mM NaCl, und wenn im Schritt a1.2) das enzymatische Aufschließen der weißen Blutkörperchen in einer Lösung mit einer Endkonzentration von 200 µg/ml Proteinase K, 10 mM Tris pH 7,6, 10 mM EDTA pH 8,0, 50 mM NaCl und 0,2 % SDS erfolgt.

In einer Weiterbildung wird die Lösung aus Schritt a1.2) für 100-140 min, vorzugsweise 120 min bei 60-70 C, vorzugsweise bei 65 C inkubiert.

Es wurde gefunden, daß auf diese Weise die Blutzellen zuverlässig und vollständig aufgeschlossen werden können, wobei eine Beschädigung der Pilzzellen vermieden wird, so daß nach diesen Verfahrensschritten sowohl die in dem Ausgangsblut frei in Lösung befindlichen als auch die phagozytierten Pilzzellen relativ unbeschädigt vorliegen und abzentrifugiert werden können, ohne ihre DNA dabei zu verlieren.

Der Schritt b1.1) umfaßt die folgenden Schritte :
- Inkubieren der im Pellet aus Schritt a2.1) befindlichen Pilzzellen bei 90-98°C, vorzugsweise 95°C für 5-15 min, vorzugsweise 10 min, in einer Lösung mit 50 mM NaOH,
- Neutralisation mit 1 M Tris-HCl pH 7,0,
- Enzymatische Behandlung mit Zymolyase für 50-70 min, vorzugsweise 60 min bei 30-40°C, vorzugsweise 37°C,
- Proteindenaturierung durch Inkubation mit Tris/EDTA bei 60-70°C, vorzugsweise 65°C, für 10-30 min, vorzugsweise 20 min.

Es wurde gefunden, daß durch diese Verfahrensschritte ein sicheres Aufschließen der Pilzzellen mit daraufhin erfolgender vollständiger Freigabe der Pilz-DNA möglich ist, so daß die Es wurde gefunden, daß durch diese Verfahrensschritte ein sicheres Aufschließen der Pilzzellen mit daraufhin erfolgender vollständiger Freigabe der Pilz-DNA möglich ist, so daß die Pilz-DNA nunmehr frei in Lösung ist und von den Zellbruchstükken der Pilzzellen isoliert werden kann.

Dabei ist es bevorzugt, wenn in diesem Zusammenhang der Schritt b2) folgende Schritte umfaßt:
- Proteinpräzipitation mit 5 M Kaliumazetat, und
- DNA-Präzipitation des Überstandes in eiskaltem Isopropanol.

Diese Verfahrensschritte sind sehr einfach durchzuführen, zunächst wird durch Zugabe von Kaliumazetat das Protein herausgefällt, dann der Überstand abgenommen und mit eiskaltem Isopropanol versetzt, so daß die DNA ausfällt. Diese ausgefallene DNA kann dann für die weiteren Verfahrensschritte herangezogen werden, also zum Nachweis und zur Identifizierung einer Pilzinfektion dienen.

Bei dem beschriebenen Verfahren ist also zusammengefaßt als erstes von Vorteil, daß die Pilzspezies sozusagen an Hand ihrer DNA allgemein nachgewiesen und dann speziell identifiziert werden kann. Die Pilz-DNA wird dabei nicht direkt aus dem Vollblut extrahiert, sondern zunächst erfolgt eine Trennung der Pilzzellen von zellulärer DNA, um die Sensitivität und Spezifität des Nachweises zu erhöhen. Mit anderen Worten, wenn nur sehr wenige Pilzzellen in dem Vollblut vorhanden sind, so könnte die daraus extrahierte sehr geringe Pilz-DNA-Menge ggf. vor dem Hintergrund der in sehr hoher Konzentration vorliegenden zellulären DNA nicht nachgewiesen werden, so daß die erwähnte Trennung hier große Vorteile bezüglich der Sensitivität bringt.

Ein weiterer Vorteil bei dem neuen Verfahren liegt darin, daß auch phagozytierte Pilzzellen für den Nachweis zur Verfügung stehen, da zunächst die Blutzellen des Vollblutes aufgeschlossen werden, ohne daß dabei die Pilzzellen, seien sie frei in Lösung oder in phagozytiertem Zustand, beschädigt werden. Erst nach Abtrennung der Pilzzellen von der zellulären DNA und den verbleibenden Zelltrümmern der Blutzellen werden dann die Pilzzellen aufgeschlossen. Das hierzu verwendete Verfahren ist in der Lage, trotz der sehr komplexen Pilzzellwände für einen vollständigen Aufschluß zu sorgen, ohne daß dabei die Pilz-DNA zerstört wird.

Beschrieben wird ebenfalls ein Kit zur Durchführung des oben beschriebenen Verfahrens. Ein derartiges Kit kann eine Zusammenstellung sämtlicher Stammlösungen enthalten, wie sie für die genannten Verfahrensschritte im einzelnen benötigt werden. Es ist jedoch auch möglich, in diesem Kit nur die nicht üblicherweise in einem Labor vorrätigen Stammlösungen vorzusehen, so daß bspw. auf die Tris-Puffer etc. verzichtet werden kann, aber zumindest die Proteinase K und die Zymolyase in dem Kit vorhanden sind.

In den Beispielen 6 bis 9 ist beschrieben, wie zunächst nachgewiesen wird, ob in dem Präzipitat überhaupt Pilz-DNA vorhanden ist, woraufhin dann das Präzipitat noch einzelnen Pilzspezies zugeordnet werden muß. Dabei wird ausgenutzt, daß die im Sequenzprotokoll unter SEQ ID-No: 1 und SEQ ID-No: 2 angegebenen DNA-Sequenzen spezifisch an Bindungsstellen auf dem Pilz-Gen für die 18 ssu-rRNA vieler Pilzstämme und -spezies binden und als Primer in einer Polymerase-Kettenreaktion Verstärkungsprodukte mit einer Länge von ca. 500 Basenpaaren erzeugen. Diese Verstärkungsprodukte können dann im Rahmen einer sequentiellen Hybridisierung bestimmten Pilzspezies zugeordnet werden, wobei als Hybridisierungssonden die im Sequenzprotokoll angegebenen Nukleotidsequenzen SEQ ID-No: 3 bis SEQ ID-No: 8 verwendet werden, die Spezies-spezifische Sonden sind.

Selbstverständlich kann die Pilz-DNA auch mit anderen z.B. molekularbiologischen Methoden nachgewiesen und einzelnen Pilzspezies zugeordnet werden, die PCR mit den spezifischen Primern sowie die anschließende sequentielle Hybridisierung stellen jedoch sehr funktionssichere, einfach zu handhabende Verfahren dar.

Weitere Vorteile ergeben sich aus der nachstehenden Beschreibung.

Beispiele für die Durchführung der einzelnen Verfahrensschritte sowie die Anwendung des Verfahrens im Rahmen eines klinischen Untersuchungsprogrammes sind in der folgenden Beschreibung angegeben.

Ein besonderer Vorteil des neuen Verfahrens liegt darin, daß es mit Vollblut durchgeführt wird, daß also z.B. keine Biopsie erforderlich ist oder noch herzustellendes Serum verwendet wird. Mit dem Vollblut wird zunächst eine Trennung der Pilzzellen von zellulärer DNA vorgenommen. Dies ist erforderlich, damit die ggf. nur in geringer Menge vorliegende Pilz-DNA nicht vor dem Hintergrund der in sehr viel höherer Konzentration vorhandenen zellulären DNA nachgewiesen werden muß. Dadurch wird also die Spezifität des Nachweisverfahrens erhöht. Zu diesem Zweck werden als erstes die roten Blutkörperchen durch osmotische Hämolyse lysiert und dann die weißen Blutkörperchen enzymatisch aufgeschlossen. Diese beiden Verfahrensschritte sind so ausgewählt, daß durch sie die Pilzzellen selbst noch nicht beschädigt werden und daß ggf. phagozytierte Pilzzellen unbeschädigt wieder freigesetzt werden, so daß auch sie für den weiteren Nachweis zur Verfügung stehen.

### Beispiel 1: Lysis roter Blutkörperchen durch osmotische Hämolyse.

Die Lysis der roten Blutkörperchen erfolgt mit Hilfe einer hypotonen Lösung. Dazu wird folgender Puffer mit folgenden Endkonzentrationen verwendet:

| | |
|---|---|
| Tris pH 7,6 | 10 mM |
| MgCl₂ | 5 mM |
| NaCl | 10 mM |

Die Lösung wird für 10 min bei Raumtemperatur inkubiert, und danach zentrifugiert.

Für diesen ersten Schritt ist eine Menge von 3 ml Vollblut ausreichend.

### Beispiel 2: Enzymatisches Aufschließen weißer Blutkörperchen

Die weißen Blutkörperchen, die ggf. Pilzzellen enthalten, werden vorsichtig aufgebrochen, indem die Zellen mit Proteinase K (200 µg/ml) der Firma Boehringer, Mannheim in folgendem Puffer mit den angegebenen Endkonzentrationen enzymatisch angedaut werden, in dem das Pellet aus Beispiel 1 aufgenommen wird:

| | |
|---|---|
| Tris pH 7,6 | 10 mM |
| EDTA pH 8,0 | 10 mM |
| NaCl | 50 mM |
| SDS | 0,2 % |
| Proteinase K | 200 µg/ml |

Dieser Puffer wird für zwei Stunden bei 65 C inkubiert.

### Beispiel 3: Trennung überwiegend intakter Pilzzellen vor allem von zellulärer DNA

Nachdem wie in den Beispielen 1 und 2 angegeben die Blutzellen aufgeschlossen wurden, so daß die zelluläre DNA freigegeben wurde, erfolgt ein Zentrifugationsschritt bei 5000 Umdrehungen/min, der zu einem erheblichen Verlust an zellulärer DNA führt, die bei dieser Zentrifugationsgeschwindigkeit nicht sedimentiert.

Im Sediment befinden sich jetzt vollständig die freien oder freigesetzten Pilzzellen, die für die weitere Verarbeitung in einem Puffer (Aqua bidest) aufgenommen werden.

### Beispiel 4: Aufschließen der Pilzzellen

Als nächstes werden die Pilzzellen alkalisch lysiert und enzymatisch behandelt, um die Pilz-DNA freizusetzen.

Dazu erfolgt zunächst eine Alkalilyse mit 200 ml 50 mM NaOH für 10 min bei 95°C.

Daraufhin erfolgt ein Neutralisationsschritt mit 1 M Tris-HCl pH 7,0.

Als nächstes werden 500 µl Zymolyase von Sigma (300 µg/ml) zugegeben und die Lösung für 60 min bei 37°C inkubiert, um die Pilzzellen enzymatisch aufzuschließen.

Daraufhin werden 500 µl Tris/EDTA und 50 µl 10%iger SDS Lösung zugegeben und das Gemisch bei 65°C für 20 min inkubiert, um das Protein zu denaturieren.

### Beispiel 5: Isolation der Pilz-DNA

In der nunmehr vorliegenden Lösung befinden sich Trümmer der Pilzzellen sowie freie Pilz-DNA, die nun isoliert werden muß. Hierzu erfolgt zunächst eine Proteinpräzipitation mit 5 M Kaliumazetat, woraufhin der Überstand abgenommen und die DNA dann durch Zugabe von eiskaltem Isopropanol ausgefällt wird. Dieses Fällungsprodukt wird dann für die weiteren Verfahrensschritte verwendet.

Durch die in den Beispielen 1 - 5 angegebenen Verfahrensschritte ist es also möglich, aus Vollblut hoch-selektiv Pilz-DNA zu extrahieren, die nun als Präzipitat vorliegt und nur gering mit zellulärer DNA verunreinigt ist, wodurch der jetzt erfolgende Nachweis sehr sensitiv und hochspezifisch erfolgen kann.

### Beispiel 6: Amplifikation eines pilzspezifischen DNA-Segmentes

In diesem Verfahrensschritt soll zunächst nachgewiesen werden, ob in dem Präzipitat aus dem Verfahrensschritt in Beispiel 5 überhaupt Pilz-DNA vorhanden ist. Dabei wird ausgenutzt, daß die im Sequenzprotokoll unter SEQ ID-No: 1 und SEQ ID-No: 2 angegebenen DNA-Sequenzen spezifisch an Bindungsstellen auf dem Pilz-Gen für die 18ssu-rRNA vieler Pilzstämme und -spezies binden.

Der Erfinder der vorliegenden Anmeldung hat nämlich erkannt, daß dieses Pilz-Gen bei den verschiedenen Pilz-Stämmen und -Gattungen ein derartiges Sequenzsegment aufweist, das einerseits von zwei Bindungsregionen für Primer flankiert wird, die für alle Pilz-Stämme und -Gattungen identisch sind, daß aber andererseits die Sequenz dieses Segmentes für die verschiedenen Pilz-Stämme und -Gattungen so verschieden ist, daß sie gleichzeitig zum Nachweis der einzelnen Pilzspezies und -Gattungen verwendet werden kann.

Die DNA-Sequenz SEQ ID-No: 1 bindet dabei an den sense-Strang, während die DNA-Sequenz SEQ ID-No: 2 an den anti-sense-Strang bindet, wobei der Abstand zwischen den beiden Bindungsstellen ca. 500 Basenpaare beträgt. Diese beiden DNA-Sequenzen SEQ ID-No: 1 und SEQ ID-No: 2 eignen sich damit als Primer für eine Polymerase-Kettenreaktion (PCR), in der folglich Verstärkungsprodukte (Amplicon) mit einer Länge von ca. 500 Basenpaaren erzeugt werden.

In der nachfolgenden Tabelle 1 ist die Lage der Primer und die Länge des jeweiligen Amplicons angegeben.

**Tabelle 1: Von Pilz-Pathogenen erhaltene Amplicons**

| Pilzspezies | Bindungsstellen der Primer Vorwärts-Primer Rückwärts-Primer | | Länge des Amplicons |
|---|---|---|---|
| | | | |
| C. albicans | bp544-563 | bp1033-1014 | bp490 |
| C. glabrata | bp546-565 | bp1047-1028 | bp502 |
| C. krusei | bp535-554 | bp1016-997 | bp482 |
| C. tropicalis | bp544-563 | bp1030-1011 | bp487 |
| C. parapsilosis | bp544-563 | bp1033-1014 | bp490 |
| A. fumigatus | bp544-563 | bp1046-1027 | bp503 |
| A. niger | bp483-502 | bp986 - 967 | bp503 |
| A. flavus | bp483-502 | bp985 - 966 | bp502 |
| A. terreus | bp481-500 | bp984 - 965 | bp503 |
| A. nidulans | bp481-500 | bp984 - 965 | bp503 |

Durch diese beiden Primer SEQ ID-No: 1 und SEQ ID-No: 2 lassen sich also für alle relevanten Pilzspezies der Gattungen Candida und Aspergillus durch das PCR-Verfahren Amplicons von ca. 500 Basenpaaren in ausreichender Menge herstellen.

Die PCR-Bedingungen sind dabei die folgenden:

| | |
|---|---|
| Puffer (50 µl): | |
| 10 mM Tris pH 9,6 | |
| 50 mM NaCl | |
| 10 mM mgCl₂ | |
| 0,2 mg/ml BSA | |
| Polymerase | |
| je 0,5 mM Nukleotide | |
| je 100 pM Primer | |
| Anfängliche Denaturierung: | 3 min bei 94 C |
| Zyklus-Denaturierung: | 0,5 min bei 94 C |
| Annealing: | 1 min bei 62 C |
| Extension: | 2 min bei 72 C |
| | Terminale Extension: |
| | 5 min bei 72 C |
| Zykluszahl: | 34 |

Die hohe Magnesiumkonzentration im Puffer sorgt für eine hohe Spezifität der Polymerase, die mit 72 C im Extensions-Schritt bei ihrem Temperaturoptimum arbeiten kann.

Mit diesen Primern konnten insgesamt 40 Stämme von Candida albicans, 10 Stämme von Candida tropicalis, 6 Stämme von Candida parapsilosis, 11 Stämme von Candida glabrata, 8 Stämme von Candida krusei, 8 Stämme von Aspergillus fumigatus, 6 Stämme von Aspergillus flavus, 5 Stämme von Aspergillus terreus, 7 Stämme von Aspergillus niger, 5 Stämme von Aspergillus nidulans und 3 Stämme von Aspergillus versiculor erfolgreich amplifiziert werden.

### Beispiel 7: Nachweis der Amplifikationsprodukte aus Beispiel 6

Im nächsten Schritt soll jetzt nachgewiesen werden, ob bei der PCR-Reaktion aus Beispiel 6 auch tatsächlich DNA-Segmente mit einer Länge von ca. 500 Basenpaaren hochverstärkt wurden. Diese Detektion der pilzspezifischen DNA-Segmente erfolgt durch Ethidiumbromid-Färbung der spezifischen Bande in einem 2%igen Agarose-Gel.

Ist hier die spezifische Bande zu erkennen, so kann von einer Pilzinfektion ausgegangen werden, da die Primer SEQ ID-No: 1 und SEQ ID-No: 2 an alle oben erwähnten Pilzstämme binden. Sollte also durch die Verfahrensschritte aus den Beispielen 1 - 5 Pilz-DNA extrahiert worden sein, so wird sie durch den PCR-Schritt des Beispiels 6 so weit hochverstärkt, daß sie hier durch Ethidiumbromid-Färbung nachgewiesen werden kann.

### Beispiel 8: Zuordnung der Amplifikationsprodukte aus Beispiel 6 zu einzelnen Pilzspezies

Für eine spezifische Therapie ist es jetzt noch erforderlich, die im Schritt 7 bereits nachgewiesene Pilzinfektion genauer zu spezifizieren. Hier zeigt sich jetzt ein weiterer Vorteil des PCR-Schrittes aus Beispiel 6. Dort wurde nämlich so viel pilzspezifisches DNA-Segment erzeugt, daß jetzt weitere Nachweisverfahren zur Bestimmung der Pilzspezies möglich sind.

Hierzu werden die im Sequenzprotokoll angegebenen Nucleotidsequenzen SEQ ID-No: 3 bis SEQ ID-No: 8 herangezogen, die als Spezies-spezifische Sonden dienen, die mit einem Sequenzabschnitt des in Beispiel 6 erzeugten DNA-Segmentes spezifisch hybridisieren.

Es wurde gefunden, daß die Sonde SEQ ID-No: 3 mit Candida albicans, SEQ ID-No: 4 mit Candida glabrata, SEQ ID-No: 5 mit Candida krusei, SEQ ID-No: 6 mit Candida tropicalis, SEQ ID-No: 7 mit Candida parapsilosis und SEQ ID-No: 8 mit Aspergillus fumigatus, A. flavus, A. versiculor, A. niger, A. nidulans und A. terreus hybridisieren. Die Nucleotidsequenz SEQ ID-No: 8 ist also eine allgemeine Aspergillus-Sonde, während die Nucleotidsequenzen SEQ ID-No: 3 - SEQ ID-No: 5 Pilzspezies der Gattung Candida voneinander unterscheiden können.

Um die erfolgte Hybridisierung nachweisen zu können, werden die Sonden mit dem Transferase-Kit der Firma Boehringer, Mannheim mit Digoxigenin markiert, wobei der Nachweis nach dem Southern Blot-Verfahren mit der üblichen Farbreaktion erfolgt.

Mit Hilfe dieser Sonden erfolgt eine sequentielle Hybridisierung, die nach der Häufigkeit der einzelnen Pilzspezies gestaffelt ist, so daß zunächst mit SEQ ID-No: 3 (C. albicans), dann mit SEQ ID-No: 8 (Aspergillus), SEQ ID-No: 6 (C. tropicalis), SEQ ID-No: 7 (C. parapsilosis), SEQ ID-No: 4 (C. glabrata) und schließlich mit SEQ ID-No: 5 (C. krusei) hybridisiert wird.

Auf diese Weise ist es also möglich, an Hand der im Schrittbeispiel 6 erzeugten Amplifikationsprodukte durch sequentielles Hybridisieren die Pilzspezies zu identifizieren und anschließend eine gezielte Therapie einzuleiten.

### Beispiel 9: Nachweis ausgesäter Pilzzellen in Blut

Mit Hilfe der spezifischen Amplifikation und sequentiellen Hybridisierung gelang es, Pilzspezies mit einer Sensitivität von 1 - 3 Pilzzellen reproduzierbar nachzuweisen. Durch Aussaat von Pilzspezies in Blutproben konnte nachgewiesen werden, daß das obige Verfahren eine Sensitivität von einer sog. CFU (colony forming unit)/ml-Blut erreicht. Diese Nachweisgrenze liegt weit unterhalb derjenigen, die bei einer klinisch relevanten Pilzaussaat in Blut erwartet werden kann.

### Beispiel 10: Klinisches Untersuchungsprogramm

In einem groß angelegten klinischen Untersuchungsprogramm konnte eine hohe Spezifität des neuen Verfahrens bei der Analyse von 165 Blutproben von 65 gesunden Probanden gezeigt werden. Alle 165 Blutproben sind negativ geblieben.

94 immunsupprimierte Patienten mit unklarem Fieber wurden daraufhin auf die Präsenz einer Pilzinfektion untersucht. Von 69 Patienten, bei denen keine invasive Pilzinfektion vorlag, waren weit über 200 Blutproben (bis auf eine Ausnahme) ebenfalls negativ.

Dagegen konnten alle 25 Patienten mit invasiver Pilzinfektion bereits innerhalb der ersten Woche als positiv identifiziert werden. Bei allen Patienten gelang darüber hinaus die Zuordnung zu dem verursachenden Pilzerreger.

### SEQUENZPROTOKOLL

ALLGEMEINE ANGABEN:
   ANMELDER:
      NAME: Eberhard-Karls-Universität Tübbingen, Universitätsklinikum
      STRASSE: Geissweg 3
      ORT: Tübingen
      LAND: Deutschland
      POSTLEITZAHL: 72076
      TELEFON: 07071-29-1 TELEFAX: 07071-293966
   BEZEICHNUNG DER ERFINDUNG: Extraktion von Pilzzellen-DNA
   ANZAHL DER SEQUENZEN: 8
   COMPUTERLESBARE FASSUNG:
      DATENTRÄGER: (folgt)
      COMPUTER:
      BETRIEBSSYSTEM:
      SOFTWARE:
   DATEN DER JETZIGEN ANMELDUNG:
      ANWALTSAKTE: 5402P155EP
   ANGABEN ZU SEQ ID-NO:1:
      SEQUENZCHARAKTERISTIKA:
         LÄNGE: 20 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:1:
         ATTGGAGGGC AAGTCTGGTG 20
   ANGABEN ZU SEQ ID-NO:2:
      SEQUENZCHARAKTERISTIKA:
         LÄNGE: 20 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:2:
         CCGATCCCTA GTCGGCATAG 20
   ANGABEN ZU SEQ ID-NO:3:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:3:
         TCTGGGTAGC CATTTATGGC GAACCAGGAC 30
   ANGABEN ZU SEQ ID-NO:4:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:4:
         TTCTGGCTAA CCCCAAGTCC TTGTGGCTTG 30
   ANGABEN ZU SEQ ID-NO:5:
      SEQUENZCHARAKTERISTIKA:
         LÄNGE: 30 Basenpaare
         ART: Nucleinsäure
         STRANGFORM: Einzelstrang
         TOPOLOGIE: linear
         HYPOTHETISCH: JA
      SEQUENZBESCHREIBUNG: SEQ ID-NO:5:
         GTCTTTCCTT CTGGCTAGCC TCGGGCGAAC 30
   ANGABEN ZU SEQ ID-NO:6:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:6:
         GTTGGCCGGT CCATCTTTCT GATGCGTACT 30
   ANGABEN ZU SEQ ID-NO:7:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:7:
         TTTCCTTCTG GCTAGCCTTT TTGGCGAACC 30
   ANGABEN ZU SEQ ID-NO:8:
      SEQUENZCHARAKTERISTIKA:
         LÄNGE: 30 Basenpaare
         ART: Nucleinsäure
         STRANGFORM: Einzelstrang
         TOPOLOGIE: linear
      HYPOTHETISCH: JA
      SEQUENZBESCHREIBUNG: SEQ ID-NO:8:
         CATGGCCTTC ACTGGCTGTG GGGGGAACCA 30

## Patentansprüche

1. Verfahren zum Nachweisen von Pilzzellen in klinischem Material unter Extrahieren von Pilz-DNA aus Vollblut, mit den Schritten:
a) Isolation überwiegend intakter Pilzzellen aus dem Vollblut, und
b) Extrahieren von DNA aus den isolierten Pilzzellen durch
b1) Aufschließen der isolierten Pilzzellen, und
b2) Isolation der Pilz-DNA,
wobei der Schritt a) die weiteren Schritte umfasst:
a1) Aufschließen der im Vollblut befindlichen Blutzellen; und
a2) Isolation überwiegend intakter Pilzzellen von zellulärer DNA
**dadurch gekennzeichnet, dass** der Schritt b1) den weiteren Schritt umfasst:
b1.1) alkalisches Lysieren und enzymatische Behandlung der Pilzzellen, wobei der Schritt a) die weiteren Schritte umfasst:
a1.1) Lysis der roten Blutkörperchen durch osmotische Hämolyse;
a1.2) enzymatisches Aufschließen der weißen Blutkörperchen; und
a2.1) Zentrifugation des so behandelten Vollblutes und Verwendung des Pellet in den nächsten Verfahrensschritten
und wobei der Schritt b1.1) folgende Schritte umfasst:
- Inkubieren der im Pellet aus Schritt a2.1) befindlichen Pilzzellen bei 90-95°C, vorzugsweise 95°C für 5-15 min, vorzugsweise 10 min, in einer Lösung mit 50 mM NaOH, und
- Neutralisation mit 1 M Tris-HC1 pH 7,0,
- enzymatische Behandlung mit Zymolase für 50-70 min, vorzugsweise 60 min bei 30-40°C, vorzugsweise 37°C,
- Proteindenaturierung durch Inkubation mit Tris/EDTA bei 60-70°C, vorzugsweise 65°C für 10-30 min, vorzugsweise 20 min.

2. Verfahren nach Anspruch 1, **dadurch gekennzeichnet, dass** im Schritt a1.1) die Lysis der roten Blutkörperchen mit Hilfe einer hypotonen Lösung und einem Detergenz erfolgt, vorzugsweise mit einer Endkonzentration von 10 mM Tris pH 7,6,5 mM MgCl₂ und 10 mM NaCl.

3. Verfahren nach Anspruch 1 oder 2, **dadurch gekennzeichnet, dass** im Schritt a1.2) das enzymatische Aufschließen der weißen Blutkörperchen in einer Lösung mit einer Endkonzentration von 200 µg/ml Proteinase K, 10 mM Tris pH 7,6, 10 mM EDTA pH 8,0, 50 mM NaCl und 0,2 % SDS erfolgt.

4. Verfahren nach Anspruch 3, **dadurch gekennzeichnet, dass** die Lösung für 100-140 min, vorzugsweise 120 min bei 60-70°C, vorzugsweise bei 65°C inkubiert wird.

5. Verfahren nach einem der Ansprüche 1 bis 4, **dadurch gekennzeichnet, dass** der Schritt b2) folgende Schritte umfasst:
- Proteinpräzipitation mit 5 M Kaliumazetat, und
- DNA-Präzipitation des Überstandes in eiskaltem Isopropanol.

## Claims

1. A method for the detection of fungal cells in clinical material by extracting fungal DNA from whole blood, comprising the steps of:
a) isolation of predominantly intact fungal cells from whole blood; and
b) extraction of DNA from the isolated fungal cells by
b1) disintegration of the isolated fungal cells, and
b2) isolation of the fungal DNA,
wherein step a) comprises the further steps:
a1 disintegration of the blood cells present in whole blood; and
a2) isolation of predominantly intact fungal cells from cellular DNA,
**characterized in that** step b1) comprises the further step of:
b1.1) alkaline lysis and enzymatic treatment of the fungal cells,
wherein step a) comprises the further steps:
a1.1) lysis of the erythrocytes by osmotic hemolysis;
a1.2) enzymatic digestion of the leukoctyes; and
a2.1) centrifugation of the whole blood thus treated and use of the pellet in the next procedural steps,
and wherein step b1.1) comprises the following steps:
- incubation of the fungal cells present in the pellet from step a2.1) at 90-95°C, preferably 95°C, for 5-15 min, preferably 10 min, in a solution with 50 mM NaOH, and
- neutralization with 1 M Tris-HC1 pH 7.0,
- enzymatic treatment with Zymolyase for 50-70 min, preferably 60 min, at 30-40°C, preferably 37°C,
- protein denaturation through incubation with Tris/EDTA at 60-70°C, preferably 65°C, for 10-30 min, preferably 20 min.

2. The method according to claim 1, **characterized in that** the lysis of the erythrocytes in step a1.1) is performed using a hypotonic solution and a detergent, preferably with a final concentration of 10 mM Tris pH 7.6, 5 mM MgCl₂ and 10 mM NaCl.

3. The method according to claim 1 or 2, **characterized in that** the enzymatic disintegration of the leukocytes in step a1.2) is performed in a solution with a final concentration of 200 µg/ml proteinase K, 10 mM Tris pH 7.6, 10 mM EDTA pH 8.0, 50 mM NaCl and 0.2% SDS.

4. The method according to claim 3, **characterized in that** the solution is incubated for 100-140 min, preferably 120 min, at 60-70°C, preferably at 65°.

5. The method according to any one of claims 1 to 4, **characterized in that** step b2) comprises the following steps:
- protein precipitation with 5 M potassium acetate, and
- DNA precipitation of the supernatant in ice-cold isopropanol.

## Revendications

1. Procédé pour la mise en évidence de cellules fongique dans un matériel clinique avec extraction d'ADN fongique du sang total, comportant les étapes:
a) isolement de cellules fongiques pour la plupart intactes à partir du sang total et
b) extraction d'ADN à partir des cellules fongiques isolées par
b1) désagrégation des cellules fongiques isolées et
b2) isolement de l'ADN fongique,
l'étape a) comportant les autres étapes:
a1) désagrégation des cellules sanguines qui se trouvent dans le sang total et
a2) isolement de cellules fongiques pour la plupart intactes d'ADN cellulaire,
**caractérisé en ce que** l'étape b1) comprend l'étape supplémentaire suivante:
b1.1) lyse alcaline et traitement enzymatique des cellules fongiques, l'étape a) comprenant les autres étapes:
a1.1) lyse des globules rouges par hémolyse osmotique;
a1.2) ésagrégation enzymatique des globules blancs; et
a2.1) centrifugation du sang total ainsi traité et utilisation du culot dans les étapes du procédé suivantes,
l'étape b1.1) comprenant les étapes suivantes:
- incubation des cellules fongique qui se trouvent dans le culot provenant de l'étape a2.1) à 90-95°C, de préférence 95°C pendant 5 à 15 min, de préférence 10 min, dans une solution avec NaOH 50 mM, et
- neutralisation avec Tris-HCl pH 7,0 1 M,
- traitement enzymatique avec la zymolase pendant 50-70 min, de préférence 60 min à 30-40°C, de préférence 37°C,
- dénaturation des protéines par incubation avec Tris/EDTA à 60-70°C, de préférence 65°C pendant 10-30 min, de préférence 20 min.

2. Procédé selon la revendication 1, **caractérisé en ce que**, dans l'étape a1.1), la lyse des globules rouges a lieu à l'aide d'une solution hypotonique et d'un détergent, de préférence avec une concentration finale de 10 mM de Tris pH 7,6, 5 mM de MgCl₂ et 10 mM de NaCl.

3. Procédé selon la revendication 1 ou 2, **caractérisé en ce que**, dans l'étape a1.2), la désagrégation enzymatique des globules blancs a lieu dans une solution avec une concentration finale de 200 µg/ml de protéinase K, 10 mM de Tris pH 7,6, 10 mM d'EDTA pH 8,0, 50 mM de NaCl et 0,2 % de SDS.

4. Procédé selon la revendication 3, **caractérisé en ce que** la solution est incubée pendant 100-140 min, de préférence 120 min à 60-70°C, de préférence à 65°C.

5. Procédé selon l'une des revendications 1 à 4, **caractérisé en ce que** l'étape b2) comprend les étapes suivantes :
- précipitation de protéines avec de l'acétate de potassium 5 M et
- précipitation de l'ADN du surnageant dans de l'isopropanol glacé.
